# EUROPEAN PATENT APPLICATION

(11) **EP 0 734 732 A1**
(43) Date of publication of application: **02.10.1996**
(21) Application number: 96200872.8
(22) Date of filing: 29.03.1996
(51) Int. Cl.: A61L 2/18

(54) **A composition for cleaning contact lenses and a method for cleaning contact lenses**

(30) Priority: 31.03.1995 NL 9500637
(71) Applicant: A.C. Obbens Beheer B.V., 2411 WP Bodegraven (NL)
(72) Inventor: Tuyl, Bernardus, 2225 BH Katwijk (NL); Wagenaar, Louis Johan, 2225 BH Katwijk (NL); Obbens, Albert Cornelis, 2411 WP Bodegraven (NL)
(74) Representative: Van kan, Johan Joseph Hubert, Ir.

(57) **Abstract**

A composition for cleaning contact lenses, which composition contains a component for decomposing hydrogen peroxide and which furthermore contains additives which are known per se as well as a colorant. The colorant added is a substance which will turn the fluid yellow. This substance is a riboflavin compound and can correspond with the formula given on the formula sheet. The composition can be brought in tablet form. The invention also concerns the method for cleaning a contact lens.

## Description

The invention relates to a composition for cleaning contact lenses, which composition contains a component for decomposing hydrogen peroxide and which furthermore contains additives which are known per se as well as a colorant. The invention furthermore relates to a method for cleaning contact lenses by using a composition according to the invention.

From EP-A-82 798 a method is known for cleaning contact lenses by immersing the contaminated contact lens into a hydrogen peroxide solution and subsequently immersing the cleaned lens in a second step into a solution containing a substance which decomposes any hydrogen peroxide still adhering to the lens into water and oxygen. In that case catalase is used as the substance which is conducive to the decomposition of hydrogen peroxide. Furthermore the solution contains additives such as common salt in order to obtain a physiological saline solution. From the said patent application it is furthermore known to form catalase and the like substances into a tablet, which tablet may be dissolved in water to produce a solution for carrying out the final cleaning step.

Meanwhile it has been found that besides catalase also other enzyms may be used for decomposing hydrogen peroxide, or that said decomposition may take place by using substances other than catalase.

Furthermore it has become apparent that the method for cleaning the contact lenses can be carried out more safely by showing the user clearly to what stage the cleaning process has progressed, since the solution of hydrogen peroxide and the solution of the hydrogen peroxide decomposing substance are both colourless. In this framework it has been proposed in W093/04706 to add an active amount of vitamin B-12 to the calase-containing solution, as a result of which the solution will be coloured red.

It has now been found that the use of a particular composition will result in a fluid, which will have a positive effect on the eye, in particular on the cornea which composition is characterized by the fact that the colorant to be added is a substance which will turn the fluid yellow.

The colorant used according to the invention is preferably a riboflavin compound especially a substance that corresponds with the formula given on the formula sheet. Such a substance is known as the sodium salt of riboflavin-5'-phosphate (vitamin B2). According to the invention this colorant is used in an amount of about 0.075 mg per tablet to be used for an amount of fluid of about 0.8 ml.

As such the use of riboflavin in eye drops is known from Chemical Abstracts vol. 97 no. 18, abstract no. 150668.

The substance according to the invention providing the yellow colour hardly adheres to the contact lens to be cleaned, if at all. In the case that some colorant has adhered to the lens, albeit in an amount which is hardly visible, said colorant may be washed away with tap water or be removed by immersion in tap water. If necessary the decoloration can take place within a few minutes in a physiological saline solution. Thus the use of the colorant according to the invention does not have a negative effect on the contact lens, whilst the use of the fluids is facilitated.

As soon as the tablet according to the invention has been added to the fluid, the concentration of the colorant will be such that 8 ml of fluid will contain about 0.075 mg of colorant, which concentration is considerably lower than in those cases where said substance (vitamin B2) is prescribed as a therapeutic agent for eyestrain.

The method for preparing the tablet is carried out in a conventional manner, whereby the colorant may be added to the additives which are normally used.

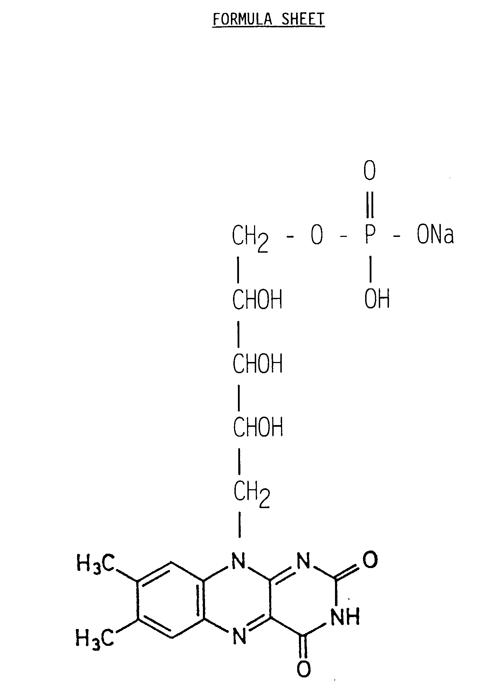

## Claims

1. A composition for cleaning contact lenses, which composition contains a component for decomposing hydrogen peroxide and which furthermore contains additives which are known per se as well as a colorant, characterized in that the colorant to be added is a substance which will turn the fluid yellow.

2. The composition according to claim 1, characterized in that the colorant is a riboflavin compound.

3. The composition according to claims 1-2, characterized in that the colorant corresponds with the formula given on the formula sheet.

4. The composition according to claim 1, characterized in that the colorant is the sodium salt of riboflavin-5'-phosphate (vitamin B2).

5. The composition according to claims 1-4, characterized in that this composition is brought into a tablet form.

6. The composition according to claim 5, characterized in that the colorant is used in the amount of about 0.075 mg (+/- 10%) per tablet as used for an amount of fluid of about 8 ml.

7. A method for cleaning a contact lens by immersing the contact lens in a hydrogen peroxide solution, after which any hydrogen peroxide adhering to the lens is decomposed in a fluid as disclosed in claims 1-4 or by adding to a solution a tablet as disclosed in claims 5-6.
